(19) **Europäisches Patentamt**

**European Patent Office**

**Office européen des brevets**

(11) Veröffentlichungsnummer: **0 572 922 A1**

## (12) EUROPÄISCHE PATENTANMELDUNG

(21) Anmeldenummer: **93108574.0**

(22) Anmeldetag: **27.05.93**

(51) Int. Cl.5: **A61K 31/385**, A61K 31/19, A61K 31/07, A61K 31/355, A61K 31/375, A61K 31/455, A61K 31/51, A61K 31/68

(30) Priorität: **05.06.92 DE 4218572**

(43) Veröffentlichungstag der Anmeldung:
**08.12.93 Patentblatt 93/49**

(84) Benannte Vertragsstaaten:
**AT BE CH DE DK ES FR GB GR IE IT LI LU MC NL PT SE**

(71) Anmelder: **ASTA Medica Aktiengesellschaft
An der Pikardie 10
D-01277 Dresden(DE)**

(72) Erfinder: **Weischer, Carl-Heinz, Dr.
Schmidtbonnstrasse 8
W-5300 Bonn 1(DE)**
Erfinder: **Ulrich, Heinz, Dr.
Bayernstrasse 2
W-8751 Niedernberg(DE)**
Erfinder: **Wessel, Klaus, Dr.
Homburger Landstrasse 46 C
W-6000 Frankturt/M. 50(DE)**

(54) **Synergistische Kombination von Liponsäure und Vitaminen in Arzneimitteln.**

(57) Verwendung von alpha-Liponsäure, Dihydroliponsäure, Metaboliten der alpha-Liponsäure u.a. 6,8-Bisnortetraliponsäure, Tewtranorliponsäure, optische Isomere R-und S- Form der alpha-Liponsäure in oxidierter und reduzierter Form in Kombination Mit Vitamin A, B1, B2, B6, B12, C und E und deren pharmazeutisch verwendbare Salze zur Herstellung von Arzneimitteln mit analgetischer, antiphlogistischer, antidiabetischer, zytoprotektiver, antiulzerativer, antinekrotischer, neuroprotektiver, detoxifizierender, antiischämischer, leberfunktionsregulierender, antiallergischer, immunstimulierender sowie antionkogener Wirkung, entsprechende Arzneimittel und deren Herstellung.

EP 0 572 922 A1

Synergistische Kombination von Arzneimitteln enthaltend als Wirkstoff alpha-Liponsäure, Dihydroliponsäure, deren Metaboliten sowie die oxidierten und reduzierten Enantiomere der alpha-Liponsäure wie die R-alpha-Liponsäure oder S-alpha-Liponsäure sowie Metaboliten der alpha-Liponsäure mit den Vitaminen A, B 1-6, B12, C, und E.

alpha-Liponsäure ist chemisch 1,2-Dithia-cyclopentan-3-valeriansäure.

alpha-Liponsäure ist in Form des R-Enantiomeren in Pflanzen und Tieren weit verbreitet; sie wirkt in vielen enzymatischen Reaktionen als Coenzym, stellt einen Wachstumsfaktor für manche Bakterien und Protozoen dar und wird bei der Behandlung von Knollenblätterpilzvergiftungen eingesetzt. Weiterhin weist das alpha-Liponsäure-Racemat antiphlogistische, antinociceptive (analgetische) sowie zytoprotektive, neuroprotektive, antiallergische und antitumor Eigenschaften auf.

Von dem reinen optischen Isomeren der alpha-Liponsäure (R-und S-Form, das heißt R-alpha-Liponsäure und S-alpha-Liponsäure) sind im Gegensatz zu dem Racemat das R-Enantiomer vorwiegend antiphlogistisch und das S-Enantiomer vorwiegend antinociceptiv wirksam, wobei ebenfalls die antiphlogistische Wirkung des R-Enantiomeren beispielsweise um einen Faktor 10 stärker ist als diejenige des Racemats.

Die antinociceptive (analgetische) Wirkung des S-Enantiomeren ist beispielweise um einen Faktor bis 6 stärker als diejenige des Racemats.

Die Enantiomeren stellen daher im Vergleich zu dem Racemat sehr viel spezifischere und stärker wirksame Wirkstoffe dar.

Diese Wirkungen sind in EP-A 901213405 beschrieben.

Der Kombinationspartner Vitamin A ist essentiell für das Wachstum, für die Knochenentwicklung, die normale Funktion der Fortpflanzungsorgane und der Augen und vor allem für die Struktur und Funktion von Schleimhautepithelien.

Der Kombinationspartner Vitamin E erhält essentiell die Funktion aller Zellen, auch der des Nervensystems. Seine wesentliche Funktion ist der Schutz von Lipiden vor der Peroxidation. Die japanische Offenlegungsschrift 3-193778 beschreibt Ester der Liponsäure mit Tocopherolen. Diese Tocopherolester der Liponsäure werden zur Behandlung von UV-Erythemen eingesetzt.

Der Kombinationspartner Vitamin B1 hat seine Wirkung hauptsächlich bei dem Thiaminmangelsyndrom, das heißt bei scherer Mangelernährung, langfristiger parenteraler Ernährung, Null-Diät, Hämodialyse und Malabsorption und Alkholabusus.

Der Kombinationspartner Vitamin B2 hat seine Wirkung hauptsächlich bei den Mangelerscheinungen, die durch Alkoholabusus und oder unzureichender Zufuhr von Milch und Milchprodukten hervorgerufen werden.

Der Kombinationspartner Vitamin B6 hat seine Wirkung hauptsächlich bei den Mangelerscheinungen, die durch Alkoholabusus oder chronische Medikamenteneinnahme (orale Kontrazeptiva, Isoniazid) hervorgerufen werden.

Der Kombinationspartner Vitamin B12 hat seine Wirkung hauptsächlich bei strengen Vegetariern, nach Magenresektionen und Atrophie der Schleimhaut oder intestinalen Erkrankungen, die zu Mangelerscheinungen wie zum Beispiel Anämien, Ausfällen des peripheren und zentralen Nervensystems und Polyneuro-pathien führen.

Der Kombinationspartner Vitamin C gehört zu den biochemischen Redoxsystemen und ist an Zahlreichen Elektronentransportreaktionen beteiligt. Hierzu zählen unter anderem die Kollagensynthese, die Noradrenalin-, Dopamin- und Serotoninsynthese und der Abbau von 4-Hydroxyphenylpyruvat. Ferner begünstigt es die Eisenresorption und übt einen stimmulierenden Effekt auf die Phagozytoseaktivität der Leukozyten aus. Neben den Carotinoiden, Vitamin A und E ist für Vitamin C eine Antitumowirkung belegt.

Aufgabe der Erfindung ist die Bereitstellung von verbesserten Arzneimitteln mit analgetischer, antiphlogistischer, antidiabetischer, zytoprotektiver, antiulzerativer, antinekrotischer, neuroprotektiver, detoxifizierender, Schwermetallantidot-, antiischämischer, leberfunktionregulierender, antiallergischer, immunstimulierender sowie antionkogener Wirkung.

Bei den für die Herstellung nach dieser Erfindung verwendeten Tocopherolen (Vitamin E) kann es sich um alpha-Tocopherol, $\beta$-Tocopherol, gamma-Tocopherol oder delta-Tocopherol handeln. Man kann diese aus natürlichen Ölen (d-Form) als auch synthetische Ware (dl-Form) verwenden.

Des weiteren können auch Tocopherolacetat oder Tocopherolacetat sowie weitere Ester physiologisch unbedenklicher Säuren verwendet werden.

Es wurde überrachend gefunden, daß in der Kombination der Wirkstoffe wie beispielsweise dem Vitamin E mit den reinen optischen Isomeren der alpha-Liponsäure (R- und S-Form, das heißt R-alpha-Liponsäure und S-alpha-Liponsäure) im Gegensatz zu dem Racemat der alpha-Liponsäure alleine das R-Enantiomere antiphlogistisch und antidiabetisch, das heißt blutzuckersenkend und das S-Enantiomere in Kombination mit Vitamin E antinociceptiv wirksam ist, wobei ebenfalls überraschend die antiphlogistische Wirkung des R-Enantiomeren in Kombination mit Vitamin E beispielsweise stärker ist als diejenige des Racemats der alpha-Liponsäure. Die antinociceptive (analgetische) Wirkung des S-Enantiomeren in Kombination mit Vitamin E ist beispielsweise stärker als diejenige des Racemats der alpha-Liponsäure. Die Enantiomeren in Kombination mit Vitaminen A, B1, B2, B6, B12, C und E stellen daher im Vergleich zu dem Racemat der alpha-Liponsäure sehr viel spezifischere und stärker wirksame Wirkstoffe dar.

Im Vergleich zu der alpha-Liponsäure (Racemat) in Kombination mit den genannten Wirkstoffen des Anspruchs 2 wie beispielsweise den Vitaminen, bestehen insbesondere folgende Unterschiede: In wässrigen Lösungen werden vorsugsweise die Salze mit pharmazeutisch verwendbaren Salzbildnern verwendet.

Die Herstellung der alpha-Liponsäure, Dihydroliponsäure oder der oxidierten oder reduzierten R-alpha-Liponsäure und der S-alpha-Liponsäure oder der Metaboliten der alpha-Liponsäure sowie deren Salze in Kombination mit den genannten Vitaminen erfolgt in bekannter Weise, beziehungsweise analog hierzu (DE-OS 41 37 773).

Als Salzbildner für die Wirkstoffe des Anspruchs 1 kommen zum Beispiel übliche Basen beziehungsweise Kationen in Frage, die in der Salzform physiologisch verträglich sind. Beispiele hierfür sind: Alkali- oder Erdalkalimetalle, Ammoniumhydroxid, basische Aminosäuren wie Arginin und Lysin, Amine der Formel N R1 R2 R3 worin die Reste R1, R2 und R3 gleich oder verschieden sind und Wasserstoff, C1-C4-Alkyl oder C1-C4-Oxyalkyl bedeuten wie beispielsweise Mono- und Diethanolamin, 1-Amino-2-propanol, 3-Amino-1-propanol; Alkylenediamine mit einer Alkylenkette aus 2 bis 6-C-Atomen wie Ethylendtamin oder Hexamethylentetramin, gesättigte cyclische Aminoverbindungen mit 4 - 6 Ringkohlenstoffatomen wie Piperidin, Piperazin, Pyrrolidin, Morpholin; N-Methylglucamin, Kreatin und Trometamol.

BEFUNDE DER KOMBINATIONEN IN VERSCHIEDENEN TESTMODELLEN:

1) Analgesie

Beispielsweise zeigt das S-Enantiomer (S-alpha-Liponsäure) in Kombination mit Vitamin E im Essigsäure-Writhing-Schmerztest an der Maus und im Randall-Selitto-Entzündungsschmerztest an der Ratte eine analgetische Wirkung, die derjenigen der alpha-Liponsäure alleine (das heißt dem Racemat) oder dem Vitamin E alleine überlegen ist (perorale Applikation).

2) Antiphlogese

Beispielsweise zeigt das R-Enantiomer (R-alpha-Liponsäure) in Kombination mit Vitamin E im Carrageenin-Ödem an der Ratte eine antiphlogistische Wirkung, die derjenigen der alpha-Liponsäure (alleine) oder dem Vitamin E alleine überlegen ist (perorale Applikation).

3) Zytoprotektive, antinekrotische und antiulzerative Wirkung

Ebenso ist beispielsweise eine zytoprotektive Wirkung im Tierversuch sowohl für die oxidierten oder reduzierten R- und S-Form der alpha-Liponsäure in Kombination mit Vitamin E bereits ab einer Dosis von 20 mg/kg R- oder S-Isomer der alpha-Liponsäure in Kombination mit 50 mg/kg Vitamin E per os vorhanden.

4) Antidiabetische Wirkung

Beispielsweise zeigt das R-Enantiomer (R-alpha-Liponsäure) in Kombination mit Vitamin E im Alloxan-Diabetes Modell oder im Streptozytozin-Diabetes-Modell an der Ratte eine antidiabetische, das heißt blutzuckersenkende Wirking, die derjenigen der alpha-Liponsäure (alleine) oder dem Vitamin E alleine überlegen ist (perorale Applikation).

5) Leberstoffwechselreguliernde Wirkung

Beispielsweise zeigt das R-Enantiomer (R-alpha-Liponsäure) in Kombination mit Vitamin E an der Ratte eine Leberenzym-regulierende Wirkung, die derjenigen der alpha-Liponsäure (alleine) oder dem Vitamin E alleine überlegen ist (perorale Applikation).

6) detoxifizierende (Schwermetallantidot) Wirkung

Im Schwermetall-Intoxikationsmodell an der Ratte ist beispielsweise eine Reduzierung der Schwermetallgehalt in der Leber und somit eine detoxifizierende Wirkung sowohl für die oxidierten oder reduzierten Racemate oder R- und S-Form der alpha-Liponsäure in Kombination mit Vitamin E bereits ab einer Dosis von 30 mg/kg R- oder S-Isomer der alpha-Liponsäure in Kombination mit 50 mg/kg Vitamin E per os vorhanden.

7) immunstimulierde Wirkung

An Panleukopenie-Virus infizierten Katzen ist beispielsweise eine immunstimulierende Wirkung im Tierversuch sowohl für die oxidierten oder reduzierten R- und S-Form der alpha-Liponsäure in Kombination mit Vitamin E bereits ab einer Dosis von 35 mg/kg R- oder S-Isomer der alpha-Liponsäure in Kombination mit 50 mg/kg Vitamin E per os vorhanden.

8) wachstumshemmende Wirkung auf Retroviren

Darüberhinaus besitzen beispielsweise die R- und S-alpha-Liponsäure in Kombination mit Vitamin E überraschenderweise eine wachstumshemmende Wirkung gegen Retroviren, insbesondere das Human-Immundefizienzvirus HIV (HIV-1, HIV-2) und sind daher auch geeignet zur Behandlung von Erkrankungen, die durch solche Viren verursacht werden. So besitzen sie eine gute, wachstumshemmende Wirkung auf HIV (Typ 1 und 2), welche sich in vitro beispielsweise durch folgende virologisch-zellbiologische Testverfahren zeigen läßt:
    1. Plaque - Reduktionstest
    2. CPE - Reduktionstest
    3. Bestimmung der Reversen Transkriptase im Kulturüberstand
    4. Bestimmung des p24 Antigens im Kulturüberstand
So wird beispielsweise bei einer einmaligen Gabe von 0,035 mg/ml R- oder S-Isomer in Kombination mit 0,1 mg Vitamin E die Anzahl der infektiösen Viren (zum Beispiel HIV-1), im Zellkulturüberstand von 100 % in der positiv Kontrolle auf 0 % gesenkt. Ein virusinhibierender Effekt kann in diesem Testverfahren bereits in sehr niedrigen Dosen, beispielsweise 0,001 mg/ml nachgewiesen werden.
Als allgemeiner Dosisbereich für die Wirkung (Versuch wie oben) kommt beispielsweise in Frage:
0,0035 - 0,091 mg/ml R- oder S-Isomer in Kombination mit 0,001 - 0,01 mg/ml Viamin E , insbesondere 0,035 - 0,070 mg/ml R- oder S-Isomer in Kombination mit 0,01, - 0,1 mg/ml Vitamin E.
Für die in vitro Versuche wird der Wirkstoff oder die Kombination aus Wirkstoffen zum Beispiel in Benzylalkohol als Lösungsmittel eingesetzt.
Für die in vitro Untersuchungen des Replikationsverhaltens von Retroviren, insbesondere HIV, können zum Beispiel folgende Substrate eingesetzt werden:
    1. Virushaltiges RPMI 1640 Medium, zum Beispiel IX Flüssig 041-01875 (synthetisches Kulturmedium von Gibeo gemäß Moore, Gerner und Franklin, H.A. (1967), J.A.M.A. 199; 519) mit einer Konzentration von $2 \times 10^3$ - $1 \times 10^3$ infektiösen Einheiten (PFU)/ml
    2. Die Zellinien Jurkat Clone E6-1, Sup T1 und HeLa CT4.
Die Kombinationen der Wirkstoffe des Anspruchs 1 mit den Wirkstoffen des Anspruchs 2 zeigen an folgenden Untersuchungsmodellen eine gute gute analgetische, antiphlogistische, antiarthrotische und zytoprotektive Wirkung:
MgSO₄-Writing-Test an der Maus nach GYIRES et al. (Arch. int. pharmacodyn. therap. 267, 131-140,1984) Adjuvans-Arthritis an der Ratte nach NEWBOULD (Brit.J.Pharmacol. 21.,127-136,1963) Na-Monojodacetat-induzierte Arthrose an Ratten bzw. Hühnern nach KALBHEN, in: Arthrosis deformans, Eular-Verlag, Basel/Schweiz, 1982; Intestitinale Ulzeration an Ratten nach DEL SOLDATO (Agents and Actions 23, 1/2, 1988)
Die Kombinationen der Wirkstoffe des Anspruchs 1 mit den Wirkstoffen des Anspruchs 2 zeigen an folgenden Untersuchungsmodellen eine zytoprotektive, detoxifizierende Wirkung:
Prüfung auf akute Zelltoxizität an Mausfibroblasten L 929 o.ä.
nach: LINDL et al. in: Zell und Gewebekultur, Gustav Fischer Verlag, Stuttgart, New York, 2.Aufl.,1989, Seite 164-167.
Die Kombinationen der Wirkstoffe des Anspruchs 1 mit den Wirkstoffen des Anspruchs 2 zeigen an folgenden Untersuchungsmodellen eine gute Wirkung auf die Stoffwechselaktivität:
Prüfung zum Einfluß einer Substanz auf Stoffwechselaktivität.

nach: LINDL et al. in: Zell und Gewebekultur, Gustav Fischer Verlag, Stuttgart, New York, 2.Aufl.,1989, Seite 167-168.

Die Kombinationen der Wirkstoffe des Anspruchs 1 mit den Wirkstoffen des Anspruchs 2 zeigen an folgenden Untersuchungsmodellen eine gute wachstumshemmende Wirkung:

Prüfung auf wachstumshemmende Eigenschaften einer Substanz mit Hilfe von Maus-Fibroblasten (L929) bzw. Humanfibroblasten (MRC9) nach: LINDL et al. in: Zell und Gewebekultur, Gustav Fischer Verlag, Stuttgart, New York, 2.Aufl.,1989, Seite 162-164.

Die Kombinationen der Wirkstoffe des Anspruchs 1 mit den Wirkstoffen des Anspruchs 2 zeigen in folgendem Untersuchungsmodell an Makrophagen eine gute phagozytosehemmende Wirkung:

Prüfung der Phagozytose - Aktivität von Makrophagen, nach G.ROSSI, in: Zellkultur-Methoden, Berlin 7.-9.Okt. 1987, Hrsg. H.R. Maurer, Inst. für Pharmazie der freien Univorsität Berlin, 29.Sept. 1987, Seite 163

TOXIKOLOGIE der KOMBINATIONEN im VERGLEICH zu DEN EINZELWIRKSTOFFEN

Die akute Toxizität der R-alpha-Liponsäure beziehungsweise der S-alpha-Liponsäure an der Maus (ausgedrückt als LD50 mg/kg; Methode LITCHFIELD und WILCOXON, J. Pharmocol. Exp Ther. 95, 99 (1949) ), liegt beispielsweise oberhalb von 1000 mg/kg bei oraler Applikation.

TOXIZITÄT

alpha-Liponsäure (Racemat)

| LD50 | p.o. mg/kg | Spezies |
|---|---|---|
| | 502 | Maus, männlich |
| | 460 | Maus, weiblich |
| | 1190 | Ratte männlich |
| | 1210 | Ratte, weiblich |

Vitamin B1

| LD50 | p.o.mg/kg | i.v.mg/kg | Spezies |
|---|---|---|---|
|  | 8200-13300 | 85 - 125 | Maus |
|  | 12300 | 200 - 250 | Ratte |

Vitamin B6

| LD50 | p.o.mg/kg | i.v.mg/kg | Spezies |
|---|---|---|---|
|  | 8400 | 1020 | Maus |
|  | 5500-15900 | 1450 | Ratte |

Vitamin B12

| LD50 | p.o. | Spezies |
|---|---|---|
|  | 1600 | Maus |

Vitamin C

| LD50 | p.o.mg/kg | i.v.mg/kg | Spezies |
|---|---|---|---|
|  | 8021 | 1058 | Maus |
|  | >5000 | 1000 | Ratte |

Vitamin E

LD50                    p.o.mg/kg              i.v.mg/kg          Spezies

                        >50.000                >2100              Maus

                        >5000                  >1500              Ratte

Beispiele: TOXIKOLOGIE DER KOMBINATIONEN

alpha-Liponsäure (Razemat) mit 30 mg/kg Vitamin E

LD50              p.o.                                            Spezies

        >1200 mg für die alpha-Liponsäure                        Maus

Vitamin E (30mg/kg p.o.) mit R-Isomer der
alpha-Liponsäure

LD50              p.o.                                            Spezies

        >1200 mg/kg für das R-Isomer der                         Maus
        alpha-Liponsäure

Vitamin E (30mg/kg p.o.) mit S-Isomer der
alpha-Liponsäure

LD50              p.o.                                            Spezies

        >1200 mg/kg für das S-Enantiomer                         Maus
        der alpha-Liponsäure

PHARMAZEUTISCHE ZUBEREITUNGEN

    Die pharmazeutischen Zubereitungen der Kombination der Wirkstoffe des Anspruchs 1 mit den Wirkstoffen des Anspruchs 2 enthalten im allgemeinen zwischen 1 mg bis 3 g als Einzeldosis, vorzugsweise

7

2 mg bis 1,2 g R-beziehungsweise S-alpha-Liponsäure beispielsweise in Kombination mit 1 bis 450 mg Vitamin E. Die erreichten Wirkspiegel/kg Körpergewicht sollen für die R- oder S-alpha-Liponsäure in der Kombination zwischen 1,5 und 200 mg, vorzugsweise zwischen 4 und 100 mg, besonders zwischen 8 und 70 mg/kg für die R- oder S-Form der alpha-Liponsäure und beispielsweise für das Vitamin E vorzugsweise zwischen 0,01 und 20 mg/kg KG, besonders zwischen 0,1 und 8 mg/kg Körpergewicht liegen.

Die Verabriechung kann beispielsweise in Form von Tabletten, Kapseln, Pillen, Dragees, Aerosolen oder in flüssiger Form erfolgen.

Als flüssige Anwendungsformen kommen zum Beispiel in Frage:
alkoholische beziehungsweise wässrige Lösungen sowie Suspensionen und Emulsionen.

Bevorzugte Anwendungsformen sind zum Beispiel Tabletten, die zwischen 10 mg und 2000 mg oder Lösungen, die zwischen 10 ml bis 0,2 g/ml Flüssigkeit aktive Substanzen enthalten.

## Tabelle 1

Beispiel für die oralen Dosen für die Therapie der Schwermetallintoxikation beim Menschen

| Substanz des Anspruchs 1 | Substanz des Anspruchs 2 | Tagesdosis der Substanz des Anspruchs 1 | Tagesdosis der Substanz des Anspruchs 2 | Einzeldosen der Substanz des<br>a) Anspruchs 1<br>b) Anspruchs 2 | Applikations-häufigkeit |
|---|---|---|---|---|---|
| Vitamin A | oxid/reduz. Racemat oder R- oder S-Isomer der alpha-Lipon-säure | 5.000 - 150.000 IE | 300 mg - 1,2 g | a) 1.000 IE - 30.000 IE<br>b) 100 mg - 400 mg | 1 - 4 |
| Vitamin B1 | oxid/reduz. Racemat oder R- oder S-Isomer der alpha-Lipon-säure | 5 - 50 mg | 300 mg - 1,2 g | a) 1 mg - 12 mg<br>b) 100 mg - 400 mg | 1 - 4 |
| Vitamin B6 | oxid/reduz. Racemat oder R- oder S-Isomer der alpha-Lipon-säure | 5 - 50 mg | 300 mg - 1,2 g | a) 1 mg - 12 mg<br>b) 100 mg - 400 mg | 1 - 4 |

| Substanz des Anspruchs 1 | Substanz des Anspruchs 2 | Tagesdosis der Substanz des Anspruchs 1 | Tagesdosis der Substanz des Anspruchs 2 | Einzeldosen der Substanz des a) Anspruchs 1 b) Anspruchs 2 | Applikations-häufigkeit |
|---|---|---|---|---|---|
| Vitamin B12 | oxid/reduz. Racemat oder R- oder S-Isomer der alpha-Lipon-säure | 5 - 50 mikro-gramm | 300 mg - 1,2 g | a) 1 mikrogramm - 12 mikrogramm b) 100 mg - 400 mg | 1 - 4 |
| Vitamin C | oxid/reduz. Racemat oder R- oder S-Isomer der alpha-Lipon-säure | 200 - 1000 mg | 300 mg - 1,2 g | a) 50 mg - 250 mg b) 100 mg - 400 mg | 1 - 4 |
| Vitamin E | Racemat oder R- oder S-Isomer der alpha-Lipon-säure | 100 - 800 mg | 250 mg - 1,2 g | a) 25 mg - 200 mg b) 60 mg - 400 mg | 1 - 4 |

Die Einzeldosis an Wirkstoff des Kombinationspartners nach Anspruch 1 in der Kombination beispielsweise mit Vitamin E kann beispielsweise liegen:

a) bei oraler Arzneiform zwischen 50 mg - 3 g, vorzugsweise 100 mg - 1,2 g.

b) bei parenteralen Arzneiformen (zum Beispiel intravenös, intramuskulär) zwischen 50 mg - 2 g, vorzugsweise 100 mg - 3 g.

10

c) bei Arzneiformen zur Inhalation (Lösungen oder Aerosole) zwischen 100 mikrogramm - 2 g, vorzugsweise 200 Mikrogramm - 1,2 g. Die Dosen gemäß a) bis c) können beispielsweise 1-bis 6mal, vorzugsweise 1- bis 4mal täglich oder aber als Dauerinfusion, beispielsweise mit Hilfe eines Infusoniaten verabreicht werden.

Beispielsweise kann die tägliche Dosis an R- beziehungsweise S-alpha-Liponsäure in der Kombination beipielsweise mit Vitamin E beim Menschen 2-40 mg pro kg Gewicht liegen; die Einzeldosis zum Beispiel bei 1-10 mg pro kg Gewicht, wobei diese Dosis zweckmäßig bis zu 4mal verabreicht wird pro Tag.

Vorzugsweise kann beispielsweise die Tagesdosis 100 - 600 mg betragen: Die Arzneimittel enthalten daher vorzugsweise 100 - 600 mg an R- beziehungsweise S-alpha-Liponsäure in einer galenischen Formulierung, wobei eine solche Dosis vorzugsweise bis zu 4mal verabreicht wird.

Für die Behandlung können zum Beispiel 3 mal täglich 1 bis 4 Tabletten mit einem Gehalt von 10 mg bis 2 g wirksamer Substanz des Anspruchs 1 oder beispielsweise bei intravenösen Injektion 1 bis 4 mal täglich eine Ampulle/Infusionsflasche von 1 bis 100 ml Inhalt mit 200 mg bis 6 g Wirksubstanz des Anspruchs 1 in Kombination mit 0,001 - 2 g Wirksubstanz des Anspruchs 2 empfohlen werden.

Bei oraler Verabreichung ist die minimale tägliche Dosis der Wirksubstanzen des Anspruchs 1 in Kombination mit Wirksubstanzen des Anspruchs 2 beispielsweise 100 mg; die maximale tägliche Dosis bei oraler Verabreichung soll 12 g nicht überschreiten.

Die Einzeldosis an Wirkstoff des Kombinationspartners nach Anspruch 2 in der Kombination mit dem R- oder S-Isomer der alpha-Liponsäure kann beispielsweise für das Vitamin E liegen:

a) bei oraler Arzneiform zwischen 10 mg - 2 g, vorzugsweise 20 mg - 800mg, insbesondere 25 mg - 300 mg.

b) bei parenteralen Arzneiformen (zum Beispiel intramuskulär) zwischen 0,1 - 25 mg/kg Körpergewicht, vorzugsweise 0,2 - 15 mg/kg Körpergewicht, insbesondere 1 - 190 mg/kg Körpergewicht.

c) bei Arzneiformen zur Inhalation (Lösungen oder Aerosole) zwischen 0,01 - 15 mg/kg Körpergewicht, vorzugsweise 0,1 mg - 10 mg/kg Körpergewicht, insbesondere 0,5 - 5 mg/kg Körpergewicht.

Die Dosen gemäß a) bis c) können beispielsweise 1 bis 6mal, vorzugsweise 1 bis 4mal täglich verabreicht werden.

Die tägliche orale Dosis an Vitamin E in Kombination mit dem oxidierten oder reduzierten Razemat oder R- oder S-Isomer der alpha-Liponsäure beim Menschen kann zum Beispiel bei 0,1 - 12 mg/kg Körpergewicht liegen; die Einzeldosis von Vitamin E in der Kombination zum Beispiel bei 0,1- 25 mg pro kg Gewicht, wobei diese Dosis zweckmäßig bis zu 4mal verabreicht wird pro Tag. Vorzugsweise beträgt die Tagesdosis von Vitamin E 200 - 800 mg: Die Arzneimittel enthalten daher vorzugsweise 10 - 250 mg an Vitamin E in einer galenischen Formulierung, wobei eine solche Dosis vorzugsweise 4mal verabreicht wird.

Für die Behandlung können zum Beispiel für das Vitamin E in der Kombination 3 mal täglich 1 bis 4 Tabletten mit einem Gehalt von 0,001 mg bis 800 mg wirksamer Substanz des Anspruchs 2 oder beispielsweise bei intravenösen Injektion 1 bis 4 mal täglich eine Ampulle/Infusionsflasche von 1 bis 100 ml Inhalt mit 0,10 mg bis 200 mg Wirksubstanz des Anspruchs 2 empfohlen werden.

Bei oraler Verabreichung ist die minimale tägliche Dosis beispielsweise für das Vitamin E in der Kombination 50 mg; die maximale tägliche Dosis bei oraler Verabreichung soll 1,5 g nicht überschreiten.

Die Arzneimittel des Anspruchs 1 und 2 können in der Humanmedizin allein oder im Gemisch mit anderen pharmakologisch aktiven Stoffen verwendet werden. Die Wirkstoffe R- beziehungsweise S-alpha-Liponsäure können auch mit jedem anderem gegen Retroviren insbesondere HIV wirksamen Mittel kombiniert werden, beispielsweise mit Didesoxyinosin, Didesoxycytiden, insbesondere aber mit alpha-Interferon und/oder Azidothymidin (AZT) oder mit Zytostatika wie beispielsweise Ifosfamid und Endoxan.

Die angegebenen Dosismengen der Wirkstoffe des Anspruchs 1 beziehen sich stets auf die freien Säuren der alpha-Liponsäure, Dihydroliponsäure oder der oxidierten oder reduzierten R- beziehungsweise S-alpha-Liponsäure. Falls diese in Form ihrer Salze verwendet werden, sind die angegebenen Dosierungen/Dosierungsbereiche dem höheren Mol-Gewicht entsprechend zu erhöhen.

Bei Kombinationen mit anderen antiretroviral-wirksamen Substanzen (Komponente b) können als Komponente b nur eine, aber auch 2 und mehr (vorzugsweise 2) antiretroviral wirksame Substanzen verwendet werden, wobei im letzteren Falle auch hier die hierfür angegebenen Dosierungen stets für die Summe der jeweils vorliegenden antiretroviralwirksamen Stoffe gelten. Der Ausdruck "Dosierungseinheit" bezieht sich stets auf eine Einzeldosis, die pro Tag auch mehrmals verabreicht werden kann.

Falls die Dosis in Form von Enzymeinheiten angegeben wird, handelt es sich um die Dosis, die für einen ganzen Tag gilt, wobei eine solche Dosis auf einmal, vorzugsweise jedoch verteilt über einen Tag (zum Beispiel in Form von Infusion) gegeben wird. Die Dosis-Angabe in Enzymeinheiten gilt insbesondere für alpha-Interferon.

Beispielsweise können für die Kombination von Vitamin E mit R- beziehungsweise S-alpha-Liponsäure mit der Komponente b zum Beispiel AZT, die beiden Komponenten jeweils zum Beispiel in einem Verhältnis von 0,01 zu 100 bis 100 zu 1 äquimolaren Anteilen wirksamer Substanz gemischt werden, insbesondere in einem Verhaltnis von 1 zu 10 bis 10 zu 1, vorzugsweise in einem Verhältnis von 0,1 zu 3 bis 3 zu 1 Anteilen. Im Falle einer Kombination von Vitamin E mit R-beziehungsweise S-alpha-Liponsäure und alpha-Interferon können die drei Komponenten zum Beispiel in folgendem Verhältnis vorliegen: 15 mg - 50 mg - 6 g R- beziehungsweise S-alpha-Liponsäure (Komponente a) zu $8 \times 10^6$ Enzymeinheiten bis $1 \times 10^5$ Enzymeinheiten alpha-Interferon, insbesondere 0,5 - 3 g Komponente a zu $1\text{-}4 \times 10^6$ Enzymeinheiten alpha-Interferon.

In der Kombination aus beispielsweise Vitamin E mit R-beziehungsweise S-alpha-Liponsäure und anderen Komponenten gemäß b) können beide Komponenten als Gemisch vorliegen. Im allgemeinen liegen die Komponenten jedoch voneinander getrennt in einer galenischen Formulierung vor, wobei hier die hierfür bekannten galenischen Formulierungen in Frage kommen: zum Beispiel eine Komponente als Tablette oder lackierte Tablette, die andere Komponente als Pulver, beide in einer Kapsel und umgekehrt eine Komponente in Form von Pellets, die andere als Pulver, Dragee oder Tablette und umgekehrt und wobei die beiden Formen zum Beispiel in einer Kapsel vorliegen; oder in Form von Mehrschichten- oder Manteltabletten. Es wird hierzu zum Beispiel auf das Buch von Karl Thoma, Arzneimittelstabilität, Frankfurt 1978, zum Beispiel Seite 207ff. verwiesen.

Die erfindungsgemäße Kombination kann aber auch als ein Erzeugnis vorliegen, bei dem jeweils die beiden Einzelwirkstoffe in vollständig voneinander getrennten Formulierungen vorliegen, wobei insbesondere die Komponente b, aber auch beide Komponenten (a und b) in Ampullen und/oder Infusionsflaschen enthalten sind, so daß auch eine getrennte oder auch zeitlich abgestufte Verabreichung möglich ist.

Falls solche vollständig getrennten Formulierungen vorliegen, sind diese aufeinander abgestimmt und enthalten die jeweiligen Wirkstoffe in der Dosierungseinheit in denselben Mengen und entsprechenden Gewichtsverhältnissen, in denen sie in der kombinierten Mischung vorliegen können.

Bei einem Erzeugnis für die getrennte Anwendung ist es auch möglich, daß beide Kombinationspartner nicht gleichzeitig verabreicht werden. In solchen Fällen kann zum Beispiel Vitamin E intramuskulär und R-beziehungsweise S-alpha-Liponsäure als Dauerinfusion gegeben werden (Dosis zum Beispiel 2 - 5 g pro Tag) und die dritte Komponente b gleichzeitig (Dosis zum Beispiel 50 - 800 mg oder $1\text{-}8 \times 10^6$ Enzymeinheiten, vorzugsweise intramuskulär) oder auch als Dauerinfusion pro Tag oder R- beziehungsweise S-alpha-Liponsäure kann zum Beispiel 4mal pro Tag gegeben werden (Einzeldosis zum Beispiel 0,5 - 2 g) und die andere Komponente b gleichzeitig (Dosis zum Beispiel 50 - 200 mg beziehungsweise $0{,}5\text{-}3 \times 10^6$ Enzymeinheiten). Es können dann zum Beispiel 1 bis 3 weitere Dosen an Komponente b (zum Beispiel zwischen 50 - 200 mg beziehungsweise $0{,}5\text{-}3 \times 10^6$ Enzymeinheiten) im Abstand von jeweils 6 und/oder 12 Stunden folgen.

Die erfindungsgemäßen Zubereitungen/Erzeugnisse können vorzugsweise auch zusätzliche Vitamine wie beispielsweise Pantothensäure und/oder Folsäure enthalten.

Zur Behandlung von Erkrankungen, die durch Retroviren, insbesondere HIV-Viren verursacht sind, sollen entsprechende Arzneimittel eine solche Menge an beispielsweise Vitamin E in Kombination mit R-beziehungsweise S-alpha-Liponsäure enthalten beziehungsweise in einer solchen Menge verabreicht werden, daß bei ein- oder mehrmaliger Applikation im Körper ein Wirkspiegel von Vitamin E zwischen 0,001 und 12 mg/kg, vorzugsweise zwischen 0,1 und 10 mg, insbesondere zwischen 0,2 und 8 mg/kg Körpergewicht vorliegen.

Als allgemeiner Dosisbereich für die Kombinationen der oben genannten Vitamine mit dem R- oder der S-alpha-Liponsäure für die analgetische Wirkung kommt beispielsweise in Frage:

0,5 - 20 mg/kg Körpergewicht oral Vitamin E in Kombination mit 1 - 100 mg/kg Körpergewicht R- oder S-Isomer der alpha-Liponsäure

Als allgemeiner Dosisbereich Kombinationen mit den oben genannten Vitaminen mit der R-alpha-Liponsäure für die antiphlogistische und zytoprotektive Wirkung kommt beispielsweise in Frage:

0,5 - 15 mg/kg Körpergewicht oral Vitamin E in Kombination mit 1 - 100 mg/kg Körpergewicht R- oder S-Isomer der alpha-Liponsäure

die detoxifizierende, Schwermetallantidot Wirkung kommt beispielsweise in Frage:

0,5 - 25 mg/kg Körpergewicht oral Vitamin E in Kombination mit 1 - 100 mg/kg Körpergewicht R- oder S-Isomer der alpha-Liponsäure

die antiallergische und immunstimulierende Wirkung kommt beispielsweise in Frage:

0,5 - 20 mg/kg Körpergewicht oral Vitamin E in Kombination mit 1 - 100 mg/kg Körpergewicht R- oder S-Isomer der alpha-Liponsäure

die Antitumor-Wirkung kommt beispielsweise in Frage:

EP 0 572 922 A1

0,5 - 25 mg/kg Körpergewicht oral Vitamin E in Kombination mit 1 - 100 mg/kg Körpergewicht R- oder S-Isomer der alpha-Liponsäure

die antidiabetische Wirkung kommt beispielsweise in Frage:

0,5 - 20 mg/kg Körpergewicht oral Vitamin E in Kombination mit 1 - 100 mg/kg Körpergewicht R- oder S-Isomer der alpha-Liponsäure

Als Indikation kommen beispielsweise in Betracht:

Entzündliche, degenerative artikuläre und extraartikuläre rheumatische Erkrankungen, nicht-rheumatische Entzündungs- und Schwellungszustände, Arthrosis deformans, Chondropathien, Periarthritiden, entzündliche und nichtentzündliche Erkrankungen der Haut, wie zum Beispiel Neurodermitis und Psoriasis, entzündliche und nicht-entzündliche Erkrankungen des Gastrointestinaltraktes, wie zum Beispiel Gastritis, Ulcus ventriculi, Ileitis, Duodenitis, Infektionen mit Campylobacter pylorici, Jejunitis, Colitis, Diabetes mellitus Typ I und II, Insulinresistenz, Polyneuropathie diabetogener, alkoholischer hepatischer und unrämischer Genese, Leberparenchym-degeneration, Hepatitis, Fettleber und Fettzirrhose, Schwermetallvergiftungen wie z.B. Kupfer, Zink, Cadmium, Nickel, Blei und Arsen sowie readioaktive Isotopenintoxikation sowie chronische Lebererkrankungen, entzündliche Atemwegserkrankungen, wie zum Beispiel Asthma bronchiale, Sarkoidose, ARDS (acute respiratory distress syndrome), degenerative Erkrankungen des ZNS, akute ischämische Zustände, Myokardinfarkt, Nierenparenchym-degeneration.

Die Tagesdosen der erfindungsgemäßen Darreichungsformen der Kombinationen für die analgetische Schwermetallantidot, detoxifizierendene, antidiabetsiche, immustimulierende beziehungsweise zytoprotektive, antinekrotische beziehungsweise antiphlogistische Wirkung bestehen zum Beispiel aus 0,01 bis 800 mg Vitamin E vorzugsweise 0,1 bis 600 mg Vitamin E in Kombination mit 0,1 bis 2000 mg, vorzugsweise 15 bis 600 mg und insbesondere 50 bis 200 mg R-alpha-Liponsäure oder S-alpha-Liponsäure.

Erfindungsgemäß kann eine Tagesdosis der Kombinationen der oben genannten Vitamine mit den optischen Isomere der alpha-Liponsäure (R-beziehungsweise S-Form jeweils) von 0,1 bis 800 mg Vitamin E vorzugsweise 1 bis 600 mg Vitamin E in Kombination mit den optischen Isomere der alpha-Liponsäure (R- oder S- Form jeweils) 10 - 600 mg, beispielsweise von 25 bis 400 mg oder 10 bis 200 mg Verabreicht werden. Die maximale Tagesdosis für die zytoprotektive Wirkung sowie für die Behandlung von Schmerz- und Entzündungszuständen soll für die Racemate oder die R- oder S-Form der alpha-Liponsäure 1,2 mg und für das Vitamin E 800 mg nicht überschreiten. Die Tagesdosen können in Form einer einmaligen Verabreichung der gesamten Menge oder in Form von 1 bis 6, insbesondere 1 - 4, Teildosen pro Tag eingesetzt werden.

Im allgemeinen ist eine Verabreichung von 1 bis 4mal, insbesondere 1 bis 3mal täglich, bevorzugt.

Beispielsweise beträgt die bevorzugte Tagesdosis in der Kombination mit Vitamin E (0,1 - 800 mg für die orale Verabreichung von Vitamin E in der Kombination und 0,1 - 20 mg/kg für die intramuskuläre Verabreichung von Vitamin E in der Kombination) sowohl für die R-alpha-Liponsäure als auch für die S-alpha-Liponsäure vorzugsweise 100 mg für die parenterale Applikationsform und 400 mg für die orale Form. Insbesondere kann beispielsweise die Tagesdosis für die parenterale Applikationsform der R- oder S-Isomere der alpha-Liponsäure in der Kombination mit Wirksubstanzen des Anspruchs 2 300 mg und 600 mg für die orale Form betragen.

Vorzugsweise werden die Arzneimittel oral verabreicht. Beispielsweise können das Vitamin E in der Kombination mit der R-alpha-Liponsäure und der S-alpha-Liponsäure insbesondere auch in Form einer Lösung appliziert werden, beispielsweise peroral, topisch, parenteral (intravenös, intraartikulär, intramuskulär, subkutan), inhalativ, transdermal. Die Arzneimittel, die als Wirkstoffe beispielsweise das Vitamin E in der Kombination mit der R-alpha-Liponsäure oder der S-alpha-Liponsäure enthalten, können zum Beispiel in Form von Tabletten, Kapseln, Pillen oder Dragees, Granulaten, Pellets, Pflaster Lösungen oder Emulsionen Formuliert werden, wobei die Wirkstoffe jeweils gegebenenfalls mit entsprechenden Hilfs- und Trägerstoffen kombiniert werden. Im falle von Lösungen jeweils) von 10 - 600 mg, beispielsweise von 25 bis 400 mg oder 10 bis 200 mg verabreicht. Die maximale Tagesdosis für die antidiabetische, immunstimulierende, antiallergische, zytoprotektive Wirkung sowie für die Behandlung von Schmerz- und Entzündungszuständen soll beispielsweise für die Kombination von Vitamin E mit der R- oder S-Isomere der alpha-Liponsäure für das Vitamin E oral 800 mg und für die R- oder S-Isomere der alpha-Liponsäure 1,2 g nicht überschreiten.

Die maximale Tagesdosis für die detoxifizierende, Schwermetallantidotwirkung Wirkung soll beispielsweise für die Kombination von Vitamin E mit der R-oder S-Isomere der alpha-Liponsäure für das Vitamin E oral 1200 mg und für die R- oder S-Isomere der alpha-Liponsäure 1200 mg nicht überschreiten.

Die Tagesdosen können in Form einer einmaligen Verabreichung der gesamten Menge oder in Form von 1 bis 6, insbesondere 1 - 4, Teildosen pro Tag eingesetzt werden. Im allgemeinen ist eine Verabreichung von 1 bis 4mal, insbesondere 1 bis 3mal täglich, bevorzugt.

13

Beispielsweise beträgt die bevorzugte Tagesdosis in der Kombination sowohl für das Vitamin E 800 mg oral vorzugsweise 600 mg oral und parenteral für Vitamin E 15 mg/kg Körpergewicht intramuskulär und für die R-alpha-Liponsäure als auch für die S-alpha-Liponsäure vorzugsweise 80 mg für die parenterale Applikationsform und 200 mg für die orale Form.

Beispielsweise können die R-alpha-Liponsäure und die S-alpha-Liponsäure in der Kombination mit beispielsweise Vitamin E insbesondere auch in Form einer Lösung appliziert werden, beispielsweise peroral, topisch, parenteral (intravenös, intraartikulär, intramuskulär, subkutan), inhalativ, transdermal. Die Arzneimittel, die als Wirkstoffe beispielsweise Vitamin E in der Kombination mit der R-alpha-Liponsäure oder die S-alpha-Liponsäure enthalten, können zum Beispiel in Form von Tabletten, Kapseln, Pillen oder Dragees, Granulaten, Pellets, Pflaster, Lösungen oder Emulsionen formuliert werden, wobei der Wirkstoff jeweils gegebenenfalls mit entsprechenden Hilfs- und Trägerstoffen kombiniert wird. Im Falle vom Lösungen enthalten diese beispielsweise 0,5 bis 20 Gewichts%, vorzugsweise 1 bis 10 Gewichts% eines der optischen Isomere der a-Liponsäure (jeweils R-Form oder S-Form) zusammen mit 0,001 bis 10 Gewichtsprozent des jeweiligen Vitamins.

Die Dosierungseinheit der Arzneimittel mit beispielsweise dem Vitamin E in Kombination mit den optischen Isomeren der alpha-Liponsäure oder einem therapeutisch verwendbaren Salz derselben (jeweils R-Form oder S-Form) kann beispielsweise enthalten:

a.) bei oralen Arzneiformen:

10 bis 1200 mg, vorzugsweise 20 bis 600 mg, insbesondere 50 bis 400 mg der optischen Isomere der alpha-Liponsäure in Kombination mit beispielsweise dem Vitamin E 0,1 bis 800 mg, vorzugsweise 1 bis 400 mg, insbesondere 1 - 300 mg.

Die Dosen können beispielsweise 1 bis 6mal, vorzugsweise 1 bis 4mal, insbesondere 1 bis 3 mal täglich verabreicht werden. Jedoch soll eine Gesamtdosis der optischen Isomere der alpha-Liponsäure von 1200 mg und beispielsweise des Vitamins E von 800 mg pro Tag für die zytoprotektive Wirkung sowie für die Behandlung von Schmerz- und Entzündungszuständen nicht überschritten werden. Dasselbe gilt auch für die folgenden unter b) bis e) aufgeführten Arzneiformen.

Außerdem soll eine Gesamtdosis der optischen R-oder S-Isomere der alpha-Liponsäure von 2000 mg und beispielsweise des Vitamins E von 1200 mg pro Tag für die detoxifizierende und Schwermetallantidot-Wirkung nicht überschritten werden.

b.) bei parenteralen Arzneiformen (zum Beispiel intravenös, intramuskulär oder intraartikulär): 10 bis 600 mg, vorzugsweise 15 bis 500 mg, insbesondere 20 bis 300 mg der optischen Isomere der alpha-Liponsäure in der Kombination besipielsweise mit Vitamin E 0,01 - 20 mg/kg Körpergewicht intramuskulär, vorzugsweise 0,1 - 12 mg/kg Körpergewicht insbesondere 1 - 10 mg/kg Körpergewicht intramuskulär.

Die Dosen können beispielsweise 1 bis 6mal, vorzugsweise 1 bis 4mal, insbesondere 1 bis 3mal täglich verabreicht werden.

c.) bei Arzneiformen zur Applikation auf die Haut und Schleimhäute (zum Beispiel als Lösungen, Lotionen, Emulsionen, Salben, Pflaster und so weiter) in der Kombination:

10 bis 500 mg R-alpha-Liponsäure oder S-alpha-Liponsäure, vorzugsweise 40 bis 250 mg, insbesondere 50 bis 200 mg mit beispielsweise dem Kombinationspartner Vitamin E 0,1 - 600 mg, vorzugsweise 1 - 400 mg, insbesondere 5 - 200 mg. Diese Dosen können beispielsweise 1 bis 6mal, vorzugsweise 1 bis 4mal, insbesondere 1 bis 3mal täglich verabreicht werden.

d.) bei Arzneiformen zur Inhalation (Lösungen oder Aerosole):

0,01 bis 300 mg, vorzugsweise 0,25 bis 150 mg, insbesondere 0,5 bis 80 mg R-alpha-Liponsäure oder S-alpha-Liponsäurein Kombination mit beispielsweise Vitamin E vorzugseise 0,001 - 20 mg/kg, insbesondere 0,01 bis 10 mg/kg. Diese Dosen können beispielsweise 1 bis 6mal, vorzugsweise 1 bis 4mal, insbesondere 1 bis 3mal täglich verabreicht werden.

Falls Lösungen verwendet werden, werden die optischen Isomere der alpha-Liponsäure und die in der Kombination enthaltenen Vitamine vorzugsweise in Form eines Salzes eingesetzt.

Selbstverständlich können auch galenische Zubereitungen hergestellt werden, welche die oben angegebenen Dosierungseinheiten 2 bis beispielsweise 6mal enthalten. Insbesondere enthalten Tabletten oder Kapseln 20 bis 800 mg der Wirdstoffe des Anspruchs 1 in Kombination mit einem Wirkstoff des Anspruchs 2, beispielsweise dem Vitamin E 1 - 1200 mg, Pellets, Pulver oder Granulate 20 bis 400 mg der Wirkstoffe des Anspruchs 1 in Kombination mit einem Wirkstoff des Anspruchs 2, beispielsweise dem Vitamin E 1 - 800 mg, Suppositorien 20 bis 300 mg der Wirkstoffe des Anspruchs 1 in Kombination mit einem Wirkstoff des Anspruchs 2, beispielsweise 1 - 600 mg dem Vitamin E R-alpha-Liponsäure oder S-alpha-Liponsäure.

Für die Bekämpfung von Retroviren (zum Beispiel AIDS) liegt die Tagesdosis zum Beispiel bei 4 - 6 g R- oder S- Isomer der alpha-Liponsäure in der Kombination mit beispielsweise dem Vitamin E 1 - 1200 mg.

Entsprechende Arzneimittel enthalten daher vorzugsweise in der Kombination mit 5 mg - 1 g Vitamin E die R-alpha-Liponsäure oder S-alpha-Liponsäure in der Einzeldosis (Dosierungseinheit) beispielsweise in einer Menge von 600 mg bis 1,5 g.

Die vorstehend angegebenen Dosierungen beziehen sich stets auf Kombinationen mit den genannten Vitaminen mit z.B. den freien optischen Isomere der alpha-Liponsäure. Falls die optischen Isomere der alpha-Liponsäure in Form eines Salzes verwendet werden, sind die angegebenen Dosierungen/Dosierungsbereiche infolge des höheren Mol-Gewichtes entsprechend zu erhöhen.

Für den Fall, däß die Kombination mit den Vitaminen wie beispielsweise dem Vitamin E mit den optischen Isomere der alpha-Liponsäure bei Tieren eingesetzt wird, kommen insbesondere folgende Indikationen in Frage: Panleukopenie, Staupe, Hepatosen, Arthrosis deforman, Arthritiden und -Dermatitiden.

Für die Behandlung von Tieren kommen zum Beispiel folgende Dosierungen (sowohl Vitamin E in Kombination mit der R-Form als auch S-Form der alpha-Liponsäure) in Frage:

Für die Behanglung von Katzen liegt die orale Einzeldosis im allgemeinen zwischen ungefähr 2 mg/kg und 50 mg/kg der Wirkstoffe des Anspruchs 1 in Kombination beispielsweise mit dem Vitamin E 0,1 bis 100 mg/kg vorzugsweise 1 bis 80 mg/kg, insbesondere 2 - 40 mg/kg Körpergewicht, die parenterale Dosis liegt zwischen 0,5 und 40 mg/kg Körpergewicht der Wirkstoffe des Anspruchs 1 in Kombination mit den Wirkstoffen des Anspruchs 2 beispielsweise dem Vitamin E 0,01 mg/kg bis 10 mg/kg, vorzugsweise 0,1 bis 8 mg/kg, insbesondere 1 - 4 mg/kg.

Für die Behanglung von Arthrosen bei Pferden und Vieh liegt die orale Einzeldosis im allgemeinen in der Kombination für die Wirkstoffe des Anspruchs 1 zwischen ungefähr 2 mg/kg und 100 mg/kg Körpergewicht und für die Wirkstoffe des Anspruchs 2 zwischen ungefähr 2 mg/kg und 100 mg/kg Körpergewicht, die parenterale Dosis in der Kombination für die Wirkstoffe des Anspruchs 1 ungefähr zwischen 0,5 und 50 mg/kg Körpergewicht und für die Wirkstoffe des Anspruchs 2 ungefähr zwischen 0,005 und 20 mg/kg Körpergewicht.

Die Wirkstoffe des Anspruchs 2 und die des Anspruchs 1 wie beispielsweise die optischen Isomere der alpha-Liponsäure sind zur Herstellung pharmazeutischer Zusammensetzungen und Zubereitungen geeignet. Die pharmazeutischen Zusammensetzungen beziehungsweise Arzneimittel enthalten beispielsweise die optischen Isomere der alpha-Liponsäure als Wirkstoff, gegebenenfalls in Mischung mit den Wirkstoffen des Anspruchs 2 oder anderen pharmakologisch beziehungsweise pharmazeutisch wirksamen Stoffen. Die Herstellung der Arzneimittel erfolgt in bekannter Weise, wobei die bekannten und üblichen pharmazeutischen Hilfsstoffe sowie sonstige übliche Träger- und Verdünnungsmittel verwendet werden können. Als derartige Träger- und Hilfsstoffe kommen zum Beispiel solche Stoffe in Frage die in folgenden Literaturstellen als Hilfsstoffe für Pharmazie, Kosmetik und angrenzende Gebiete empfohlen beziehungsweise angegeben sind:

Ullmanns Enzyklopädie der technischen Chemie, Band 4 (1953), Seite 1 bis 39; Journal of Pharmaceutical Sciences, Band 52 (1963), Seite 918 ff., H. v. Czetsch-Lindenwald, Hilfsstoffe für Pharmazie und angrenzende Gebiete; Pharm. Ind., Heft 2 (1961), Seite 72 ff.i Dr. H.P. Fiedler, Lexikon der Hilfsstoffe für Pharmazie, Kosmetik und angrenzende Gebiete, Cantor KG, Aulendorf in Württemberg (1989). Die pharmazeutische und galenische Handhabung der Vitamine des Anspruchs 2 und der Wirkstoffe des Anspruchs 1 wie beispielsweise der R- beziehungsweise S-alpha-Liponsäure erfolgt nach den üblichen Standardmethoden.

Beispielsweise werden in 1 bis 5 ml Vitamin E z.B. 250 mg Dihydroliponsäure oder in 10 ml Vitamin E z.B. 250 mg R-alpha beziehungsweise S-alpha-Liponsäure oder und Hilfs- beziehungsweise Trägerstoffe durch Rühren oder Homogenisieren (zum Beispiel mittels üblicher Mischgeräte) gut vermischt (klare Lösung), wobei im allgemeinen bei Temperaturen zwischen 20 und 50 C, vorzugsweise 20 bis 40 C, insbesondere bei Raumtemperatur gearbeitet wird. Im übrigen wird auf das folgende Standardwerk verwiesen: Sucker, Fuchs, Speiser, Pharmazeutische Technologie, Thieme-Verlag Stuttgart, 1978.

Die Applikation der Wirkstoffe des Anspruchs 2 mit den Wirkstoffen des Anspruchs 1 wie beispielsweise der R-beziehungsweise S-alpha-Liponsäure beziehungsweise der Arzneimittel kann auf die Haut oder Schleimhaut oder in das Körperinnere erfolgen, beispielsweise oral, enteral, pulmonal, nasal, lingual, intravenös, intraarteriell, intrakardial, intramuskulär, intraperitoneal, intracutan, subcutan.

Bei den parenteralen Zuberitungsformen handelt es sich insbesondere um sterile beziehungsweise sterillisierte Erzeugnisse.

Falls beispielsweise das Vitamin E in Kombination mit der R- beziehungsweise S-alpha-Liponsäure in Form ihrer Salze verwendet wird, kann der Salzbildner auch im Überschuß eingesetzt werden, das heißt in einer höheren Menge als äquimolar.

Biespiele für die Träger- und Hilfsstoffe sind Gelatine, natürliche Zucker wie Rohrzucker oder Milchzukker, Lecithin, Pektin, Stärke (zum Beispiel Malsstärke oder Amylose), Cyclodextrine und Cyclodextrinderivate, Dextran, Polyvinylpyrrolidon, Polyvinylacetat, Gummi arabicum, Alginsäure, Tylose, Talkum, Lycopodium, Kieselsäure (zum Beispiel kolloidale), Cellulose, Cellulosederivate (zum Beispiel Celluloseether, bei denen die Cellulose-Hydroxygruppen teilweise mit niederen gesättigten aliphatischen Alkoholen und/oder niederen gesättigten aliphatischen Oxyalkoholen verethert sind, zum Beispiel Methyloxypropylcellulose, Methylcellulose, Hydroxypropyl-methylcellulose, Hydroxypropylmethyl-cellulosephthalat, Fettsäuren sowie Magnesium-, Calcium- oder Aluminiumsalze von Fettsäuren mit 12 bis 22 C-Atomen, insbesondere der gesättigten (zum Beispiel Stearate), Emulgatoren, Öle und Fette, insbesondere pflanzliche (zum Beispiel Erdnußöl, Rizinusöl, Olivenöl, Sesamöl, Baumwollsaatöl, Maisöl, Weizenkeimöl, Sonnenblumensamenöl, Kabeljau-Leberöl, jeweils auch hydriert); Glycerinester und Polyglycerinester aus gesättigten Fettsäuren C12H2402 bis C18H3602 und deren Gemische, wobei die Glycerin-Hydroxy-gruppen vollständig oder auch nur teilweise verestert sind (zum Beispiel Mono-, Di- und Triglyceride) pharmazeutisch verträgliche ein- oder mehrwertige Alkohole und Polyglykole wie Polyethylenglykole (Molekulargewichtsbereich zum Beispiel 300 bis 1500) sowie Derivate hiervon, Polyethylenoxid, Ester von aliphatischen gesättigten oder ungesättigten-Fettsäuren (2 bis 22 C-Atome, insbesondere 10 - 18 C-Atome) mit einwertigen aliphatischen Alkoholen (1 bis 20 C-Atome) oder mehrwertigen Alkoholen wie Glykolen, Glycerin, Diethylenglykol, Pentaerythrit, Sorbit, Mannit und so weiter, die gegebenenfalls auch verethert sein können, Ester der Zitronensäure mit primären Alkoholen, Essigsäure, Harnstoff, Benzylbenzoat, Dioxolane, Glyzerinformale, Tetrahydrofurfurylalkohol, Polyglykolether mit C1-C12-Alkoholen, Dimethylacetamid, Lactamide, Lactate, Ethylcurbonate, Silicone (insbesondere mittelviskose Polydimethylsiloxane), Calciumcarbonat, Natriumcarbonat, Calciumphosphat, Natriumphosphat, Magnesiumcarbonat und ähnliche. Als weitere Hilfsstoffe kommen auch Stoffe in Frage, die den Zerfall bewirken (sogenannte Sprengmittel) wie: quervernotztes Polyvinylpyrrolidon, Natriumcarboxymethylstärke, Natriumcarboxymethylcellulose oder mikrokristalline Cellulose. Ebenfalls können bekannte Hüllstoffe verwendet werden. Als solche kommen beispielsweise in Frage:
Polymerisate sowie Copolymerisate der Acrylsäure und/oder Methacrylsäure und/oder deren Ester; Copolymerisate aus Acryl- und Methacrylsäureestern mit einem geringen Gehalt an Ammoniumgruppen (zum Beispiel EudragitR RS), Copolymerisate aus Acryl- und Methacrylsäusreestern und Trimethylammoniummethacrylat (zum Beispiel EudragitR RL); Polyvinylacetat; Fette, Öle, Wachse, Fettalkohole; Hydroxypropylmethylcellulosephthalat oder -acetatsuccinat; Cellulose-acetatphthalat Stärke-acetatphthalat sowie Polyvinylacetatphthalat; Carboxymethylcellulose; Methylcellulosephthalat, Methylcellulosesuccinat, -phthalatsuccinat sowie Methylcellulose-phthal-säurehalbester; Zein; Ethylcellulose sowie Ethylcellulosesuccinat; Schellack, Gluten; Ethylcarboxyethylcellulose; Ethacrylat-Maleinsäureanhydrid-Copolymer; Maleinsäurenanhydrid-Vinylmethylether-Copolymer; Styrol-Maleinsäure-Copolymerisate; 2-Ethyl-hexyl-acrylatmaleinsäureanhydrid; Crotonsäure-Vinylacetat-Copolymer; Glutaminsäure/Glutaminsäureester-Copolymer; Carboxymethylethylcelluloseglycerinmono-octanoat; Celluloseacetatsuccinat; Polyarginin. Als Plastifizierungsmittel für Hüllstoffe können in Frage kommen:
Citronen- und Weinsäureester (Acetyltriethylcitrat, Acetyltributyl-, Tributyl-, Triethyl-citrat); Glycerin und Glycerinester (Glycerindiacetat,-triacetat, acetylierte Monoglyceride, Rizinusöl); Phthalsäureester (Dibutyl-, Diamyl-, Diethyl-, Dimethyl-, Dipropyl-phthalat), Di-(2-Methoxy- oder 2-ethoxyethyl)-phthalat, Ethylphthalylglycolat, Butylphthalylethylglycolat und Butyglycolat; Alkohole (Propylenglycol, Polyethylenglycol verschiedener Kettenlängen), Adipate (Diethyl-adipat, Di-(2-Methoxy-oder 2-ethoxyethyl)-adipat); Benzophenon; Diethyl- und Dibutylsebacat, Dibutylsuccinat, Dibutyltartrat; Diethylenglycoldipropionat; Ethylenglykoldiacetat, -dibutyrat, -dipropionat; Tributylphosphat, -Tributyrin, Polyethylenglykolsorbitanmonooleat (Polysorbate wie Polysorbat 80); Sorbitanmonooleat. Zur Herstellung von Lösungen oder Suspensionen kommen beispielsweise Wasser oder physiologisch verträgliche organische Lösungsmittel in Frage, wie zum Beispiel Alkohole (Ethanol, Propanol, Isopropanol, 1,2-Propylenglykol, Polyglykole und deren Derivate, Fettalkohole, Partialester des Glycerins), Öle (zum Beispiel Erdunßöl, Olivenöl, Sesamöl, Mandelöl, Sonnenblumen-öl, Sojabohnenöl, Ricinusöl), Paraffine, Dimethylsulfoxid, Triglyceride und ähnliche. Für injizierbare Lösungen oder Suspensionen kommen zum Beispiel nicht-toxische parenteral verträgliche Verdünnungsmittel oder Lösungsmittel in Frage, wie zum Beispiel: Wasser, 1,3-Butandiol, Ethanol, 1,2-Propylenglykol, Polyglykole in Mischung mit Wasser, Glycerol, Ringer's Lösung, isotonische Kochsalzlösung oder auch gehärtete Öle einschließlich synthetischer Mono- oder Diglyceride oder Fettsäuren wie Oleinsäure.

Bei der Herstellung der Zubereitungen können bekannte und übliche Lösungsvermittler, beziehungsweise Emulgatoren, verwendet werden. Als Lösungsvermittler und Emulgatoren kommen beispielsweise in Frage: Polyvinylpyrrolidon, Sorbitanfettsäureester wie Sorbitantrioleat, Phosphatide, wie Lecithin, Acacia, Traganth, polyoxytheyliertes Sorbitanmonooleat und andere ethoxylierte Fettsäureester des Sorbitan, polyoxyethylierte Fette, polyoxyethylierte Oleotriglyceride,-linolisierte Oleotriglyceride, Polyethylenxoyd-Kondensationsprodukte von Fettalkoholen, Alkylphenolen oder Fettsäuren oder auch 1-Methyl-3-(2-hydroxyethyl) imidazolidon-(2).

Polyoxyethyliert bedeutet hierbei, daß die betreffenden Stoffe Polyoxyethylenketten enthalten, deren Polymerisationsgrad im allgemeinen zwischen 2 bis 40 und insbesondere zwischen 10 bis 20 liegt. Solche polyoxyethylierten Stoffe können beispielsweise durch Umsetzung von hydroxygruppenhaltigen Verbindungen (beispielsweise Mono- oder Diglyceride oder ungesättigte Verbindungen wie zum Beispiel solchen, die Ölsäurereste enthalten) mit Ethylenoxyd erhalten werden (zum Beispiel 40 Mol Ethylenoxyd pro 1 Mol Glycerid). Beisplele für Oleotri-glyceride sind Olivenöl, Erdnußöl, Rizinusöl, Sesamöl, Baumwollsaatöl, Maisöl.

Siehe auch Dr. H. P. Fledler "Lexikon der Hilfsstoffe für Pharmazie, Kosmetik und angrenzende Gebiete" 1971, S. 191-195.

Darüberhinaus ist der Zusatz von Konservierungsmitteln, Stabilisatoren, Puffersubstanzen, Geschmackskorrigentien, Süßbmitteln, Farbstoffen, Antioxydantien und Komplexbildnern und dergleichen möglich. Als Komplexbildner kommen beispielsweise in Frage: Chelatbildner wie Ethylendiamino-tetraessigsäure, Nitrilotriessigsäure, Diethylentriaminpentaessigsäure sowie deren Salze. Weiterhin kommen als Komplexbildner auch solche in Frage, die Wirkstoffe des Anspruchs 2 in Kombination mit beispeilsweise der R- oder S-alpha-Liponsäure in einem Hohlraum einschließen.

Beispiele hierfür sind Harnstoff, Thioharnstoff, Cyclodextrine, Amylose. Gegebenenfalls ist zur Stabilisierung des Wirkstoffmoleduls mit physiologisch verträglichen Basen oder Puffern auf einen pH-Bereich von ca. 6 bis 9 einzustellen. Im allgemeinen wird ein möglichst neutraler bis schwach basischer (bis pH 8) pH-Wert bevorzugt.

Als Antioxydantien kommen beispeilsweise Natriumsulfit, Natriumhydrogensulfit, Natriummetabisulfit, Ascorbinsäure, Ascorbylpalmitat, -myristat, -stearat, Gallussäure, Gallussäurealkylester, Butylhydroxyanisol, Nordihydroguajaretsäure, Tocopherole sowie Synergisten (Stoffe, die Schwermetalle durch Komplexbildung binden, beispielsweise Lecithin, Ascorbinsäure, Phosphorsäure Ethylendiaminotetra-essigsäure, Citrate, Tartrate) zur Anwendung. Der Zusatz der Synergisten steigert die antioxygene Wirkung der Antioxydantien erheblich.

Als Konsevierungsmittel kommen beispielsweise Sorbinsäure, p-Hydroxybenzoesäureester (zum Beispiel Niederalkylester), Benzoesäure, Natriumbenzoat, Trichlorisobutylalkohol, Phenol, Kresol, Benzethoniumchlorid, Chlorhexidin und Formalinderivate in Betracht.

## Beispiel 1

Suppositorien mit 50 mg Dihydroliponsäure oder mit R- bzw. S-alpha-Liponsäure und 200 mg alpha-Tocopherol bzw. 200 mg alpha-Tocopherolacetat

5 g Ascorbylpalmitat und 5 g Oxynex LM**)(E. Merck, Darmstadt) werden In 175 g geschmolzenem Hartfett**)suspendiert. Anschließend werden 20 g alpha-Tocophorol und 5 g Dihydroliponsäure zugemischt und die Mischung nach Homogenisierung in Hohlzellen zu 2,3 ml ausgegossen und abgekühlt. Vor dem Verschließen werden die Hohlzelien mit Stickstoff begast.

Ein Suppositorium vom Gewicht 2,1 g enthält 50 mg Dihydroliponsäure und 200 mg alpha-Tocopherol.

In gleicher Weise können Suppositorien mit R- Oder S-$\alpha$-Liponsäure hergestellt werden, wenn statt Dihydroliponsäure die gleiche Menge entweder R- Oder S-$\alpha$-Liponsäure eingesetzt wird.

## Beispiel 2

Kapseln mit 200 mg Dihydroliponsäure oder mit R- bzw. S-alpha-Liponsäure und 500 mg alpha-Tocopherol bzw. alpha-Tocopherolacetat

200 g R-alpha-Liponsäure werden mit 500 g alpha-Tocopherol vermischt. Anschließend werden 595 g Miglyol$^R$-Neutralöl und 100 g Sorbitsirup, 25 g Glycerol hinzugemischt und die Mischung In Kapseln der

**) Oxynex LM ist ein handelsüblicher Zusatzstoff für Fette und fetthaltige Lebensmittel. Er stellt eine hellbraune bis braune, wachsartige Masse dar, die beim Erwärmen auf 55°C zu einer klaren braunen Flüssigkeit schmilzt und enthält -Tocopherol, Ascorbylpalmitat, Citronensäure und Lecithin.

*) Hartfett ist ein Gemisch von Mono-, Di- und Triglyceriden der gesättigten Fettsäuren von $C_{10}H_{20}O_2$ bis $C_{18}H_{36}O_2$

•) Miglyol$^R$ist ein handelsübliches Gemisch von mittelkettigen Triglyceriden

Größe 00 gegeben. Eine Kapsel vom Gewicht 1,42 g enthält 200 mg R- bzw. S-alpha-Liponsäure und 500 mg alpha-Tocopherol.

In gleicher Weise können Kapseln mit Dihydroliponsäure oder mit S-α-Liponsäure hergestellt werden, wenn statt R-α-Liponsäure die gleiche Menge entweder Dihydroliponsäure oder S-α-Liponsäure eingesetzt wird.

**Beispiel 3**

Ampullen mit 250 mg R- bzw. S-α-Liponsäure und 250 mg Vitamin C (Ascorbinsäure) in 10 ml

250 g R-α-Liponsäure werden zusammen mit 352,3 g Trometamol (2-Amino-(hydroxymethyl) - 1,3-Propandiaol) in einer Mischung aus 8 Liter Wasser für Injektionszwecke und 200 g 1,2-Prophylenglykol unter Rühren gelost. Anschließend werden 250 g Vitamin C in diesem Ansatz gelöst. Die Lösung wird mit Wasser für Injektionszwecke auf 10 Liter aufgefüllt und anschließend durch ein Membranfilter der Porenweite 0,2 μm mit Glasfaservorfilter filtriert. Das Filtrat wird unter aseptischen Bedingungen zu 10 ml in sterilisierte 10 ml-Ampullen abgefüllt.

Eine Ampulle enthält in 10 ml Injektionslösung 250 mg R-α-Liponsäure als Trometamolsalz und 250 mg Vitamin C.

In gleicher Weise können Ampullen mit S-α-Liponsäure hergestellt werden, wenn stat R-α-Liponsäure die gleiche Menge S-α-Liponsäure eingesetzt wird.

**Beispiel 4**

Tabletten mit 50 mg S- bzw. R-α-Liponsäure und 50 mg Vitamin C Ascorbinsäure.

250 g S-α-Liponsäure und 250 g Vitamin C werden mit 550 g mikrokristalliner Cellulose gleichmäßig verrieben. Nach dem Sieben der Mischung werden 250 g Stärke (Starch 1500/Colorcon), 682,5 g Lactose, 15 g Magnesiumstearat und 2,5 g hochdisperses Siliciumdioxid zugemischt und die Mischung zu Tabletten vom Gewicht 400,0 mg verpreßt.

Eine Tablette enthält 50 mg S-α-Liponsäure und 50 mg Vitamin C.

In gleicher Weise können Tabletten mit 50 mg R-α-Liponsäure hergestellt werden, wenn statt 250 g S-α-Liponsäure die gleiche Menge R-α-Liponsäure eingesetzt wird.

Gegebenenfalls können die Tabletten nach üblichen Verfahren mit einem magensaftiöslichen oder magensaftpermeablen Filmüberzug versehen werden.

**Beispiel 5**

Ampullen mit 50 mg Dihydroliponsäure oder mit 50 mg R- bzw. S-alpha-Liponsäure und mit 200 mg alpha-Tocopherolacetat in 4 ml Injektionslösung

50 g R-alpha-Liponsäure werden mit 750 g alpha-Tocopherolacetat gelöst. Die Lösung wird mit 3200 g Neutralöl aufgefüllt.

Die Lösung wird mittels gamma- oder beta-Strahlen sterilisiert und unter aseptischen Bedingungen zu 10 ml in sterilisierte 10 ml-Ampullen abgefüllt. Eine 4 ml Ampulle enthält 50 mg R-alpha-Liponsäure und 200 mg alpha-Tocopherolacetat.

In gleicher Weise können Ampullen mit Dihydroliponsäure oder mit S-α-Liponsäure hergestellt werden, wenn statt R-α-Liponsäure die gleiche Menge entweder Dihydroliponsäure oder S-α-Liponsäure eingesetzt wird.

**Beispiel 6**

Salbe mit 2% Dihydroliponsäure oder 2% R- bzw. S-alpha-Liponsäure und mit 2% alpha-Tocopherolacetat

20 g R-alpha-Liponsäure werden mit 20 g alpha-Tocopherolacetat mit 400 g Vaselinum album und 100 g Sorbitol 70% und 100 g Alcohol cetylicus et stearylicus und 360 g Wollwachs vermischt. Diese Mischung wird nach Homogenisierung unter sterilen Bedingungen in 50 g Tuben abgefüllt.

Die Salbe enthält 2% R-alpha-Liponsäure und 2% alpha-Tocopherolacetat

In gleicher Weise kann eine Salbe mit Dihydroliponsäure oder mit S-α-Liponsäure hergestellt werden, wenn statt R-α-Liponsäure die gleiche Menge entweder Dihydroliponsäure oder S-α-Liponsäure eingesetzt wird.

18

**Beispiel 7**

Tabletten mit 120 mg S- bzw. R-α-Liponsäure und 61 mg Vitamin C Ascorbinsäure.

825 g S-α-Liponsäure und 425 g Vitamin C werden mit 550 g mikrokristalliner Cellulose gleichmäßig verrieben. Nach dem Sieben der Mischung werden 250 g Stärke (Starch 1500/Colorcon), 682,5 g Lactose, 15 g Magnesiumstearat und 2,5 g hochdisperses Siliciumdioxid zugemischt und die Mischung zu Tabletten vom Gewicht 400,0 mg verpreßt.

Eine Tablette enthält 120 mg S-α-Liponsäure und 61 mg Vitamin C.

In gleicher Weise können Tabletten mit 120 mg R-α-Liponsäure heregestellt werden, wenn statt 825 g S-α-Liponsäure die gleiche Menge R-α-Liponsäure eingesetzt wird.

Gegebenenfalls können die Tabletten nach üblichen Verfahren mit einem magensaftiöslichen oder magensaftpermeablen Filmüberzug versehen werden.

Synergistische Kombination von Arzneimitteln enthaltend als Wirkstoff alpha-Liponsäure, Dihydroliponsäure, deren Metaboliten sowie die oxidierten und reduzierten Enantiomere der alpha-Liponsäure wie die R-alpha-Liponsäure oder S-alpha-Liponsäure sowie Metaboliten der alpha-Liponsäure mit den Vitaminen A, B 1-6, B12, C und E.

**Patentansprüche**

1. Arzneimittel enthaltend als Wirkstoff alpha-Liponsäure, Dihydroliponsäure und deren oxidierten oder reduzierten R- oder S-Isomeren sowie Metaboliten der Alpha-Liponsäure (die 6,8-Bisnorliponsäure und Tetranorliponsäure) und mindestens ein Vitamin beziehungsweise ein pharmazeutisch verwendbares Salz hiervon.

2. Arzneimittel nach Anspruch 1, dadurch gekennzeichnet, daß das Vitamin A, B1, B6, B12, C oder E ist und zusammen mit üblichen pharmazeutischen Trägern, Hilfsstoffen und/oder Verdünnungsmitteln enthält.

3. Arzneimittel nach Anspruch 1 und 2, dadurch gekennzeichnet, daß Lösungen, die als Wirkstoff alpha-Liponsäure, Dihydroliponsäure und deren oxidierten und reduzierten R- oder S-Isomeren sowie Metaboliten der alpha-Liponsäure enthalten, Stabilisatoren und/oder Lösungsvermittler enthalten.

4. Arzneimittel nach einem oder mehreren der vorangehenden Ansprüche, dadurch gekennzeichnet, daß in der Dosiseinheit der Kombination auf 1 Gewichtsteil alpha-Liponsäure, Dihydroliponsäure und deren oxidierten oder reduzierten R- oder S-Isomeren sowie Metaboliten der alpha-Liponsäure 0,001 bis 0,8 Gewichtsteile der Vitamine kommen.

5. Arzneimittel nach einem oder mehreren der vorangehenden Ansprüche, dadurch gekennzeichnet, daß die Kombination 5 - 6000 mg, vorzugsweise 10 - 3000 mg alpha-Liponsäure, Dihydroliponsäure und deren oxidierten oder reduzierten R- oder S-Isomeren sowie Metaboliten der alpha-Liponsäure und 0,001 - 3000 mg beziehungsweise 1 - 150.000 IE des Vitamins enthält.

6. Arzneimittel nach einem oder mehreren der vorangehenden Ansprüche, dadurch gekennzeichnet, daß die Kombination 2 - 3000 mg, vorzugsweise 2 - 1000 mg alpha-Liponsäure, Dihydroliponsäure und deren oxidierten oder reduzierten R- oder S-Isomeren sowie Metaboliten der alpha-Liponsäure und 0,001 - 1000 mg beziehungsweise 1 - 150.000 IE des Vitamins enthält.

7. Arzneimittel nach einem oder mehreren der vorangehenden Ansprüche, dadurch gekennzeichnet, daß die Kombination 2 - 1000 mg, vorzugsweise 2 - 800 mg alpha-Liponsäure, Dihydroliponsäure und deren oxidierten oder reduzierten R- oder S-Isomeren sowie Metaboliten der alpha-Liponsäure und 0,005 - 5000 mg beziehungsweise 1 - 150.000 IE des jeweilgen Vitamins (Wirkstoff gemäß Anspruch 2) enthält.

8. Arzneimittel nach Anspruch 3, dadurch gekennzeichnet, daß als Stabilisatoren beziehungsweise Lösungsvermittler folgende Stoffe verwendet werden:
aliphatische C2-C4-Alkohole, die eine, zwei oder drei Hydroxylgruppen enthalten, Polyethylenglykole mit Molgewichten zwischen 200 bis 600 übliche physiologische verträgliche organische Amide, natürliche alpha-Aminosäuren, aliphatische Amine, Hydroxyethyltheophyllin, Trometamol, Diethylenglykolmono-methylether.

9. Verwendung von Stoffen oder von Stoffgemischen gemäß einem oder mehreren der vorangegangenen Ansprüche zur Herstellung eines Arzneimittels zur Bekämpfung von Schmerz, Entzündungen, Diabetes mellitus Typ I und Typ II, ulcus Gastri, Nekrosen, neurodegenerative Erkrankungen (Alzheimer, morbus Parkinson), Neuropathien, Degenerationen des peripheren und zentralen Nervensystems, Schwermetallvergiftungen, ischämische Zustände, Leberdisfunktionen, Allergien, Immunschwäche (insbesondere durch HIV indiziert) und Tumorerkrankungen sowie zur Bekämpfung von Retroviren, insbesondere AIDS und Panleukopenie verwendet werden kann.

10. Verfahren zur Herstellung eines Arzneimittels, dadurch gekennzeichnet, daß es in der Kombination von alpha-Liponsäure, Dihydroliponsäure und deren oxidierten oder reduzierten R- oder S- Isomeren sowie Metaboliten der alpha-Liponsäure mit mindestens einem Vitamin wie zum Beispiel A, B1, B2, B6, B12, C, E oder ein pharmazeutisch verwendbares Salz hiervon mit gebräuchlichen pharmazeutischen Hilfs- und Trägerstoffen und/oder Verdünnungsmitteln beziehungsweise sonstigen Hilfsstoffen zu pharmazeutischen Zubereitungen verarbeitet beziehungsweise in eine therapeutisch anwendbare Form gebracht wird.

11. Verfahren zur Herstellung eines Arzneimittels, dadurch gekennzeichnet, daß man in der Kombination von alpha-Liponsäure, Dihydroliponsäure und deren oxidierten oder reduzierten R- oder S- Isomeren sowie Metaboliten der alpha-Liponsäure mit mindestens einem Vitamin wie zum Beispiel A, B1, B2, B6, B12, C, E oder ein pharmazeutisch verwendbares Salz hiervon zusammen mit üblichen Träger- und/oder Verdünnungs- beziehungsweise Hilfsstoffen bei Temperaturen zwischen 0°C und 120°C, vorzugsweise 20°C bis 80°C, vermischt beziehungsweise homogenisiert und gegebenenfalls die so erhaltene Mischung zur Herstellung von Zubereitungen, die in der Dosierungseinheit 1 mg bis 6000, vorzugsweise 1 bis 1000 mg der alpha-Liponsäure, Dihydroliponsäure und deren oxidierten oder reduzierten R- oder S-Isomeren sowie Metaboliten der alpha-Liponsäure in Kombination mit mindestens einem Vitamin wie zum Beispiel A, B1, B2, B6, B12, C, E, F in der Dosierungseinheit 0,001 bis 1000 mg beziehungsweise 1 - 150.000 IE oder ein pharmazeutisch verwendbares Salz der Kombinationspartner enthalten, in Hohlzellen entsprechender Größe ausgießt, zu Tabletten verpreßt oder in Kapseln ensprechender Größe abfüllt oder granuliert und dann gegebenenfalls unter Zusatz von weiteren üblichen Hilfsstoffen zu Tabletten verpreßt oder in Kapseln abfüllt.

12. Verfahren zur Herstellung eines Arzneimittels, dadurch gekennzeichnet, daß man die unter den Ansprüchen 1 und 2 aufgeführten Kombinationspartner oder ein pharmazeutisch verwendbares Salz hiervon mit einem oder mehreren der folgenden Stoffe: Stärke, Cyclodextrin, Harnstoff, Cellulose, Lactose, Formalin-Casein, modifizierte Stärke, Magnesiumstearat, Calciumhydrogenphosphat, Kieselsäure, Talkum vermischt, die erhaltene Mischung gegebenenfalls mit einer wässrigen Lösung, die als Bestandteil mindestens Gelatine, Stärke, Polyvinylpyrrolidon, vinyl-pyrrolidon-Vinylacetat-Copolymerisat und/oder Polyoxyethylensorbitanmomooleat enthält, granuliert, das Granulat gegebenenfalls mit einem oder mehreren der oben genannten Hilfsstoffe homogenisiert, und die Mischung zu Tabletten verpreßt oder in Kapseln abfüllt, wobei die Tabletten oder Kapseln in der Dosierungseinheit jeweils 1 mg bis 6000, vorzugsweise 10 bis 1000 mg Wirkstoff des Anspruch 1 in Kombination mit mindestens einem Wirkstoffen des Anspruchs 2 in der Dosierungseinheit jeweils 0,001 - 2000 mg beziehungsweise 1 - 150.000 IE oder ein Salz hiervon enthalten.

13. Verfahren zur Herstellung eines Arzneimittels, dadurch gekennzeichnet, daß man Wirkstoff des Anspruchs 1 in Kombination mit mindestens einem Wirkstoff des Anspruchs 2 oder ein pharmazeutisch verwendbares Salz hiervon bei einer Temperatur zwischen 20°C bis 120°C, gegebenenfalls in Gegewart von 0,001 Gewichsteil (bezogen auf 1 Gewichsteil der Wirkstoffe des Anspruchs 1 in Kombination mit mindestens einem Wirkstoff des Anspruchs 2) Antioxidans und oder gegebenenfalls in Gegenwart eines oder mehrerer Emulgatoren und/oder Komplexibildnern mit mindestens einem der folgenden Stoffe homogenisiert und/oder emulgiert: Wasser, Glycerin, Paraffin, Vaseline, aliphatischer Alkohol mit 12 bis 25 C-Atomen, aliphatische Monocarbonsäure mit 15 bis 20 C-Atomen, Sorbitanmono-palmitat, Polyoxyethylenpolyolfettsäureester, ein- oder mehrwertiger niedrigmolekularer aliphatischer Alkohol, Fettsäureglycerid, Wachs, Silikon, Polyethylenglykol, Polyethylenoxid.

14. Verfahren zur Herstellung eines Arzneimittels (Lösung, Emulsion, Suspension), dadurch gekennzeichnet, daß man Wirkstoff des Anspruchs 1 in Kombination mit mindestens einem Wirkstoff des Anspruchs 2 oder ein pharmazeutisch verwendbares Salz hiervon bei Temperaturen zwischen 20 und 100 C, gegebenenfalls mit 0,001 bis 1 Gewichtsteilen (bezogen auf 1 Gewichsteil der Wirkstoffe des Anspruchs 1 in Kombination mit mindestens einem Wirkstoff des Anspruchs 2 ) Antioxydans sowie gegebenenfalls in Anwesenheit eines Wasser, Komplexbildners und/oder eines Emulgators in Wasser, physiologisch unbedenklichen Alkoholen, Ölen oder Dimethylsulfoxid oder Mischungen hiervon auflöst, und gegebenenfalls die so erhaltene Lösung mit soviel Wasser, Alkohol, Dimethylsulfoxid oder Öl auffüllt, daß die Endlösung, Endsuspension oder Emulsion 0,5 - 20 Gewichtsprozent an Wirkstoff der Ansprüche 1 und 2 enthält.

15. Verwendung von Wirkstoffen des Anspruchs 1 in Kombination mit mindestens einem Wirkstoff des Anspruchs 2 und deren pharmazeutisch verwendbaren Salzen zur Herstellung von Arzneimitteln.

16. Verwendung von Wirkstoffen des Anspruchs 1 in Kombination mit mindestens einem Wirkstoff des Anspruchs 2 sowie deren pharmazeutisch verwendbare Salze, dadurch gekennzeichnet, daß Arzneimittel zur Bekämpfung von Leberfunktionsstörungen, Schmerz- und Entzündungsorkrankungen,
   - Schwermetallvergiftungen insbesondere mit den Schwermetallen Kupfer, Zink, Blei, Cadmium, Nickel und Arsen
   - und/oder gleichzeitiger zytoprotektiver, antiulzerativer, antinekrotischer, antidiabetischer, neuroprotektiver und immunstimulierender Wirkung hergestellt werden.
   - und/oder gleichzeitiger antiallergischer Wirkung hergestellt werden.
   - und/oder gleichzeitiger Antitumor-Wirkung hergestellt werden.

17. Verwendung von Wirkstoffen des Anspruchs 1 in Kombination mit mindestens einem Wirkstoff des Anspruchs 2 sowie deren pharmazeutisch verwendbare Salze, dadurch gekennzeichnet, daß Arzneimittel zur Bekämpfung von Erkrankungen, die durch Retroviren: insbesondere Panleukopenie oder HIV-Viren verursacht sind, hergestellt werden.

**Europäisches Patentamt**

# EUROPÄISCHER RECHERCHENBERICHT

Nummer der Anmeldung

EP 93 10 8574
Seite 1

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| X | DE-U-9 200 543 (RABIEN M.)<br>7. Mai 1992<br>siehe das ganze Dokument<br>--- | 1,2,9,10 | A61K31/385<br>A61K31/19<br>A61K31/07<br>A61K31/355 |
| P,X | Week 2393,<br>Derwent Publications Ltd., London, GB;<br>AN 93-174387<br>& SU-A-1 738 285 (NON-BLACK SOIL AREA VETERINARY INST.) 7. Juni 1992<br>--- | 1,2 | A61K31/375<br>A61K31/455<br>A61K31/51<br>A61K31/68 |
| X | EP-A-0 127 012 (SUNTORY)<br>5. Dezember 1984<br>siehe Ansprüche 10 und 11, Tabellen 6-9*<br>--- | 1,2,9,10 | |
| X | DIALOG INFORMATION SERVICES, FILE 155 MEDLINE, ACCESSION NO 01011565;<br>& SOV. MED.<br>Bd. 30, Nr. 1, Januar 1967,<br>Seiten 86 - 89<br>ROZANOVA LB ET AL. '(Experience with the use of thioctane and galascorbin in the treatment of epidemic hepatitis)'<br>siehe den Abstract<br>--- | 1,2,9,10 | |
| X | Week 0068,<br>Derwent Publications Ltd., London, GB;<br>AN 66-17664F<br>& JP-B-40 014 867 (TAISHO PHARM)<br>* Zusammenfassung*<br>--- | 1,3 | RECHERCHIERTE SACHGEBIETE (Int. Cl.5)<br><br>A61K |
| X | FAT SCI. TECHNOL.<br>Bd. 93, Nr. 6, 1991,<br>Seiten 216 - 221<br>PRUIJN F.B. ET AL 'Interplay between Vitamin E, Glutathione and Dihydrolipoic Acid in protection against lipid peroxidation'<br>siehe die Zusammenfassung, Figur 3<br>--- | 1,2 | |

-/--

Der vorliegende Recherchenbericht wurde für alle Patentansprüche erstellt

| Recherchenort | Abschlußdatum der Recherche | Prüfer |
|---|---|---|
| MUENCHEN | 14 SEPTEMBER 1993 | B. ISERT |

Europäisches
Patentamt

**EUROPÄISCHER RECHERCHENBERICHT**

Nummer der Anmeldung

EP    93 10 8574
Seite 2

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5 ) |
|---|---|---|---|
| X | DIALOG INFORMATION SERVICES, FILE 155 MEDLINE, ACCESSION NO 07956775 & VESTN OFTALMOL Bd. 107, Nr. 3, Juni 1991, Seiten 19 - 21 FILINA AA. ET AL '(Effect of lipoic acid on tyrosine metabolism in patients with open-angle glaucoma)' siehe den Abstract ----- | 1,2 | |
| | | | **RECHERCHIERTE SACHGEBIETE (Int. Cl.5 )** |

Der vorliegende Recherchenbericht wurde für alle Patentansprüche erstellt

| Recherchenort | Abschlußdatum der Recherche | Prüfer |
|---|---|---|
| MUENCHEN | 14 SEPTEMBER 1993 | B. ISERT |